# EUROPÄISCHE PATENTSCHRIFT

(11) **EP 2 566 865 B1**
(45) Veröffentlichungstag und Bekanntmachung des Hinweises auf die Patenterteilung: **25.06.2014**
(21) Anmeldenummer: 11718702.1
(22) Anmeldetag: 02.05.2011
(51) Int. Cl.: C07D 417/14, A01N 43/78

(54) **THIAZOLDERIVATE ALS SCHÄDLINGSBEKÄMPFUNGSMITTEL**
Thiazole derivatives as pest controller
Dérivés de thiazole utilisés comme pesticides

(30) Priorität: 07.05.2010 US 332340 P; 05.05.2010 EP 10162027
(43) Veröffentlichungstag der Anmeldung: 13.03.2013
(73) Patentinhaber: Bayer Intellectual Property GmbH, 40789 Monheim (DE)
(72) Erfinder: FÜSSLEIN, Martin, 40225 Düsseldorf (DE); BRETSCHNEIDER, Thomas, 53797 Lohmar (DE); FISCHER, Reiner, 40789 Monheim (DE); JESCHKE, Peter, 51467 Bergisch Gladbach (DE); KÖHLER, Adeline, 40764 Langenfeld (DE); KLUTH, Joachim, 40764 Langenfeld (DE); MÜHLTHAU, Friedrich, August, 65812 Bad Soden am Taunus (DE); VOERSTE, Arnd, 50674 Köln (DE); SATO, Yoshitaka, Tsukuba-shi Ibaraki 3002648 (JP)
(74) Vertreter: BIP Patents
(86) Internationale Anmeldenummer: PCT/EP2011/056973
(87) Internationale Veröffentlichungsnummer: WO 2011/138285

(56) Entgegenhaltungen:
- WO-A1-03/015776
- WO-A2-2010/006713
- DE-A1- 19 725 450

## Beschreibung

Die vorliegende Anmeldung betrifft neue heterocyclische Verbindungen, Mittel enthaltend diese Verbindungen, ihre Verwendung zur Bekämpfung von tierischen Schädlingen, zu denen Arthropoden und insbesondere Insekten zählen, und Verfahren zur Herstellung der neuen Verbindungen.

Bestimmte Thiazolylverbindungen sind bereits bekannt geworden, eine Verwendung zur Bekämpfung tierischer Schädlinge wurde jedoch nicht beschrieben (vgl. WO 2003/015776 A1).

Kürzlich wurden weitere Thiazolylverbindungen bekannt, die im Pflanzenschutz Verwendung fmden können (WO 2010/006713 A1).

Moderne Planzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert wird.

Gelöst wird die Aufgabe, sowie weitere nicht explizit genannte Aufgaben, die aus den hierin diskutierten Zusammenhängen ableitbar oder erschließbar sind, durch neue Verbindungen der Formel (I) 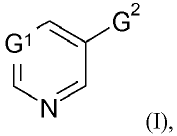 worin die Substituenten die folgenden Bedeutungen haben.
- G¹: steht für N, CH oder C-Halogen.
- G²: steht für 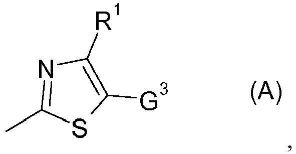 worin
R¹ für einen Rest aus der Reihe Cyano, Nitro, Fluor, Chlor, Brom und Iod steht.
G³ steht für einen Heterocyclus aus der Reihe Dioxanyl, Oxazolinyl, Dihydrooxadiazinyl, Dihydrodioxazinyl und Hydroxypyridyl, welcher substituiert ist durch einen oder mehrere Reste aus der Reihe Halogen, Cyano, Nitro, Alkyl, Halogenalkyl, Cycloalkyl, Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl, Aminocarbonyl, Pyridyl und Pyrimidyl.
- G³: steht auch für Heteroaryl aus der Reihe Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl, insbesondere Pyridyl, Pyrimidyl, Imidazolyl, Pyrazolyl, Triazinyl, Thiazolyl, Thiadiazolyl, Oxadiazolyl und Oxazolyl, welches substituiert ist durch einen Heterocyclylrest aus der Reihe Triazolinon-yl, Dihydrodioxazinyl, Dihydrooxadiazinyl, Dioxolanyl, Dioxanyl (insbesondere 1,3-Dioxan-2-yl), Piperidinonyl, Pyrrolidinonyl und Pyrazolinonyl (welcher selbst wiederum substituiert sein kann durch Alkyl und Halogenalkyl), durch Phenyl (welches selbst wiederum substituiert sein kann durch Halogen, Cyano, Nitro, Alkyl und Halogenalkyl), durch einen Heteroarylrest aus der Reihe Pyrrolyl, Pyridyl, Pyridyl-N-oxid, Pyrimidyl, Imidazolyl, Pyrazolyl, Oxazolyl, Thiazolyl, Furanyl, Thienyl, Triazolyl, Oxadiazolyl, Thiadiazolyl, Pyrazinyl, Triazinyl, Tetrazinyl und Isochinolinyl (welcher selbst wiederum substituiert sein kann durch Halogen, Nitro, Alkyl, Halogenalkyl, Alkoxy, Halogenalkoxy, Alkoxyalkyl, Alkylthio, Alkylthioalkyl und Cycloalkyl) oder durch einen Heteroarylalkylrest aus der Reihe Triazolylalkyl, Pyridylalkyl, Pyrimidylalkyl und Oxadiazolylalkyl (welcher selbst wiederum substituiert sein kann durch Alkyl).

Besonders bevorzugt sind Verbindungen der Formel (I) 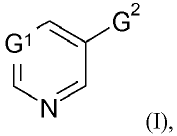 worin die Substituenten die folgenden Bedeutungen haben.
- G¹: steht für N, CH oder C-Halogen (im Fall von C-Halogen insbesondere für C-F, C-Cl oder C-Br).
- G²: steht für 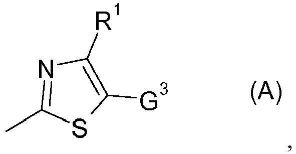 worin
R¹ für Cyano, Fluor oder Chlor steht.
G³ steht für einen Heterocyclus aus der Reihe 1,3-Dioxan-2-yl, Oxazolinyl, Dihydrooxadiazinyl, Dihydrodioxazinyl oder Hydroxypyridyl, welcher substituiert ist durch einen oder mehrere Reste aus der Reihe Halogen, Cyano, Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkylthio, C₁-C₆-Haloalkylthio, C₁-C₆-Alkylsulfinyl, C₁-C₆-Alkylsulfonyl, C₁-C₆-Haloalkylsulfinyl, C₁-C₆-Haloalkylsulfonyl, Amino, C₁-C₆-Alkylamino, Di-(C₁-C₆-alkyl)amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl, Aminocarbonyl, Pyridyl und Pyrimidyl.
- G³: steht auch für Heteroaryl aus der Reihe Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl (insbesondere für Pyridyl, Pyrimidyl, Imidazolyl, Pyrazolyl, Triazinyl, Thiazolyl, Thiadiazolyl, Oxadiazolyl und Oxazolyl), welches jeweils substituiert ist durch einen Heterocyclylrest aus der Reihe Morpholinyl, Triazolinonyl, Dihydrodioxazinyl, Dihydrooxadiazinyl, Dioxolanyl, Dioxanyl, Piperidinonyl, Pyrrolidinonyl und Pyrazolinonyl (welcher selbst wiederum substituiert sein kann durch C₁-C₆-Alkyl und C₁-C₆-Halogenalkyl), durch Phenyl (welches selbst wiederum substituiert sein kann durch Halogen, Cyano, Nitro, C₁-C₆-Alkyl und C₁-C₆-Halogenalkyl), durch einen Heteroarylrest aus der Reihe Pyrrolyl, Pyridyl, Pyridyl-N-oxid, Pyrimidyl, Imidazolyl, Pyrazolyl, Oxazolyl, Thiazolyl, Furanyl, Thienyl, Triazolyl, Oxadiazolyl, Thiadiazolyl, Pyrazinyl, Triazinyl, Tetrazinyl und Isochinolinyl (welcher selbst wiederum substituiert sein kann durch Halogen, Nitro, C₁-C₆-Alkyl, C₁-C₆-Halogenalkyl, C₁-C₆-Alkoxy, C₁-C₆-Halogenalkoxy, C₁-C₆-Alkoxy-C₁-C₆-alkyl, C₁-C₆-Alkylthio, C₁-C₆-Alkylthio-C₁-C₆-alkyl und C₃-C₆-Cycloalkyl) oder durch einen Heteroarylalkylrest aus der Reihe Triazolyl-C₁-C₆-alkyl, Pyridyl-C₁-C₆-alkyl, Pyrimidyl-C₁-C₆-alkyl und Oxadiazolyl-C₁-C₆-alkyl (welcher selbst wiederum substituiert sein kann durch C₁-C₆-Alkyl).

Ganz besonders bevorzugt sind Verbindungen der Formel (I), 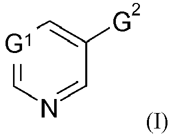 worin die Substituenten die folgenden Bedeutungen haben.
- G¹: steht für N, CH oder C-Halogen (im Fall von C-Halogen insbesondere für C-F oder C-Cl).
- G²: steht für 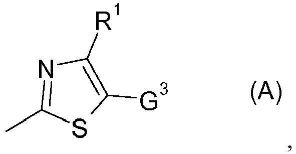 worin
R¹ für Cyano, Fluor oder Chlor steht.
G³ steht für einen Heterocyclus aus der Reihe 1,3-Dioxan-2-yl, Oxazolinyl, Dihydrooxadiazinyl, Dihydrodioxazinyl und Hydroxypyridyl, welcher substituiert ist durch einen oder mehrere Reste aus der Reihe Halogen, Cyano, Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylthio, C₁-C₄-Haloalkylthio, C₁-C₄-Alkylsulfmyl, C₁-C₄-Alkylsulfonyl, C₁-C₄-Haloalkylsulfmyl, C₁-C₄-Haloalkylsulfonyl, Amino, C₁-C₄-Alkylamino, Di-(C₁-C₄-alkyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₃-C₆-Cycloalkyl-C₁-C₄-alkyl, C₁-C₄-Alkylcarbonyl, C₁-C₄-Alkoxycarbonyl, Aminocarbonyl, Pyridyl und Pyrimidyl.
- G³: steht auch für einen Heteroarylrest aus der Reihe Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl (insbesondere Pyridyl, Pyrimidyl, Imidazolyl, Pyrazolyl, Triazinyl, Thiazolyl, Thiadiazolyl, Oxadiazolyl oder Oxazolyl), welcher substituiert ist durch einen Heterocyclylrest aus der Reihe Morpholinyl, Triazolinonyl, Dihydrodioxazinyl, Dihydrooxadiazinyl, Dioxolanyl, Dioxanyl, Piperidinonyl, Pyrrolidinonyl und Pyrazolinonyl (welcher selbst wiederum substituiert sein kann durch C₁-C₄-Alkyl und C₁-C₄-Halogenalkyl), durch Phenyl (welches selbst wiederum substituiert sein kann durch Halogen, Cyano, Nitro, C₁-C₄-Alkyl und C₁-C₄-Halogenalkyl), durch einen Heteroarylrest aus der Reihe Pyrrolyl, Pyridyl, Pyridyl-N-oxid, Pyrimidyl, Imidazolyl, Pyrazolyl, Oxazolyl, Thiazolyl, Furanyl, Thienyl, Triazolyl, Oxadiazolyl, Thiadiazolyl, Pyrazinyl, Triazinyl, Tetrazinyl und Isochinolinyl (welcher selbst wiederum substituiert sein kann durch Halogen, Nitro, C₁-C₄-Alkyl, C₁-C₄-Halogenalkyl, C₁-C₄-Alkoxy, C₁-C₄-Halogenalkoxy, C₁-C₄-Alkoxy-C₁-C₄-alkyl, C₁-C₄-Alkylthio, C₁-C₄-Alkylthio-C₁-C₄-alkyl und C₃-C₆-Cycloalkyl) oder durch einen Heteroarylalkylrest aus der Reihe Triazolyl-C₁-C₄-alkyl, Pyridyl-C₁-C₄-alkyl, Pyrimidyl-C₁-C₄-alkyl oder Oxadiazolyl-C₁-C₄-alkyl (welcher selbst wiederum substituiert sein kann durch C₁-C₄-Alkyl).

Ganz besonders hervorgehoben sind Verbindungen der Formel (I) 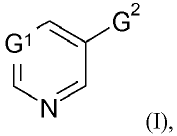 worin die Substituenten die folgenden Bedeutungen haben.
- G¹: steht für N, CH oder C-Halogen (im Fall von C-Halogen insbesondere für C-F).
- G²: steht für 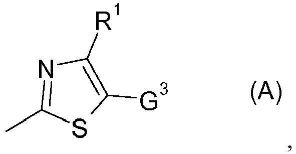 worin
R¹ für Fluor oder Chlor steht.
G³ steht für jeweils gegebenenfalls durch Pyridyl oder Pyrimidinyl substituiertes 1,3-Dioxan-2-yl, Oxazolinyl, Dihydrooxadiazinyl oder Hydroxypyridyl,
- G³: steht auch für einen Heteroarylrest aus der Reihe Pyrazolyl, 1,2,4-Oxadiazolyl, Pyridyl, Pyrimidinyl, 1,3,5-Triazinyl, Triazinyl und Oxadiazolyl, welcher substituiert ist durch einen Heterocyclylrest aus der Reihe Morpholinyl, Triazolinonyl, Dihydrodioxazinyl, Dihydrooxadiazinyl, Piperidinonyl, Pyrrolidinonyl und Pyrazolinonyl (welcher selbst wiederum substituiert sein kann durch C₁-C₄-Alkyl (insbesondere Methyl) und C₁-C₄-Halogenalkyl (insbesondere CF₃)), durch Phenyl (welches selbst wiederum substituiert sein kann durch Halogen (insbesondere Fluor, Chlor)), durch einen Heteroarylrest aus der Reihe Pyrrolyl, Pyridyl (insbesondere 2-Pyridyl), Pyridyl-N-oxid, Pyrimidinyl (insbesondere 2-Pyrimidinyl), Pyrazolyl (insbesondere 1-Pyrazolyl und 3-Pyrazolyl), Thiazolyl, Furanyl, Thienyl, Triazolyl, Oxadiazolyl, Thiadiazolyl (insbesondere 1,2,4-Thiadiazol-3-yl), Pyrazinyl, Triazinyl und Isochinolinyl (welcher selbst wiederum substituiert sein kann durch Halogen (insbesondere Fluor, Chlor), Nitro, C₁-C₄-Alkyl (insbesondere Methyl, Ethyl, n-Propyl, i-Propyl, tert.-Butyl), C₁-C₄-Halogenalkyl (insbesondere CF₃, CHF₂, CFClH), C₁-C₄-Alkoxy (insbesondere Methoxy, Ethoxy), C₃-C₆-Cycloalkyl (insbesondere Cyclopropyl, Cyclobutyl, Cyclopentyl)) oder durch einen Heteroarylalkylrest aus der Reihe Triazolyl-C₁-C₄-alkyl (insbesondere Triazolylmethyl), Pyridyl-C₁-C₄-alkyl (insbesondere Pyridylmethyl), Pyrimidinyl-C₁-C₄-alkyl (insbesondere Pyrimidinylmethyl) und Oxadiazolyl-C₁-C₄-alkyl (insbesondere Oxadiazolylmethyl (welcher selbst wiederum substituiert sein kann durch C₁-C₄-Alkyl (insbesondere Methyl)).

Durch Halogen (auch "Halo" abgekürzt) substituierte Reste, z.B. Haloalkyl (= Halogenalkyl), sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom und Iod, insbesondere für Fluor, Chlor und Brom, hervorgehoben für Fluor und Chlor.

Der Rest "Pyrimidyl" wird auch als "Pyrimidinyl" bezeichnet.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Die oben aufgeführten allgemeinen oder in Vorzugsbereiche aufgeführten Restedefinitionen bzw. Erläuterungen gelten für die Endprodukte und für die Ausgangsprodukte und Zwischenprodukte entsprechend. Diese Restedefmitionen können untereinander, also auch zwischen den jeweiligen Vorzugsbereichen, beliebig kombiniert werden.

Erfindungsgemäß bevorzugt sind Verbindungen der Formel (I), in welchen eine Kombination der vorstehend als bevorzugt aufgeführten Bedeutungen vorliegt.

Erfindungsgemäß besonders bevorzugt sind Verbindungen der Formel (I), in welchen eine Kombination der vorstehend als besonders bevorzugt aufgeführten Bedeutungen vorliegt.

Erfindungsgemäß ganz besonders bevorzugt sind Verbindungen der Formel (I), in welchen eine Kombination der vorstehend als ganz besonders bevorzugt aufgeführten Bedeutungen vorliegt.

Erfindungsgemäß ganz besonders hervorgehoben sind Verbindungen der Formel (I), in welchen eine Kombination der vorstehend als ganz besonders hervorgehoben aufgeführten Bedeutungen vorliegt.

In einer hervorgehobenen Gruppe von erfmdungsgemäßen Verbindungen der Formel (I) steht G¹ für CH.

In einer weiteren hervorgehobenen Gruppe von erfindungsgemäßen Verbindungen der Formel (I) steht G¹ für N.

In einer weiteren hervorgehobenen Gruppe von erfmdungsgemäßen Verbindungen der Formel (I) steht G¹ für C-Halogen.

In einer weiteren hervorgehobenen Gruppe von erfmdungsgemäßen Verbindungen der Formel (I) steht G³ für einen Heterocyclus.

In einer weiteren hervorgehobenen Gruppe von erfindungsgemäßen Verbindungen der Formel (I) steht G³ für Heteroaryl.

In einer weiteren hervorgehobenen Gruppe von erfindungsgemäßen Verbindungen der Formel (I) steht G¹ für CH und R¹ für Fluor.

In einer weiteren hervorgehobenen Gruppe von erfmdungsgemäßen Verbindungen der Formel (I) steht G¹ für N und R¹ für Fluor.

In einer weiteren hervorgehobenen Gruppe von erfindungsgemäßen Verbindungen der Formel (I) steht G¹ für C-Halogen und R¹ für Fluor.

In einer weiteren hervorgehobenen Gruppe von erfmdungsgemäßen Verbindungen der Formel (I) steht G¹ für CH und R¹ für Chlor.

In einer weiteren hervorgehobenen Gruppe von erfindungsgemäßen Verbindungen der Formel (I) steht G¹ für N und R¹ für Chlor.

In einer weiteren hervorgehobenen Gruppe von erfmdungsgemäßen Verbindungen der Formel (I) steht G¹ für C-Halogen und R¹ für Chlor.

In einer weiteren hervorgehobenen Gruppe von erfindungsgemäßen Verbindungen der Formel (I) steht G¹ für CH und R¹ für Cyano.

In einer weiteren hervorgehobenen Gruppe von erfmdungsgemäßen Verbindungen der Formel (I) steht G¹ für N und R¹ für Cyano.

In einer weiteren hervorgehobenen Gruppe von erfindungsgemäßen Verbindungen der Formel (I) steht G¹ für C-Halogen und R¹ für Cyano.

Die Verbindungen der Formel (I) können gegebenenfalls in Abhängigkeit von der Art der Substituenten als geometrische und/oder als optisch aktive Isomere oder entsprechende Isomerengemische in unterschiedlicher Zusammensetzung vorliegen. Die Erfindung betrifft sowohl die reinen Isomere als auch die Isomerengemische.

Beispielhaft und ergänzend wird die Herstellung von Verbindungen der Formel (I) in dem folgenden Formelschema erläutert. An dieser Stelle sei auch auf die Herstellungsbeispiele verwiesen. 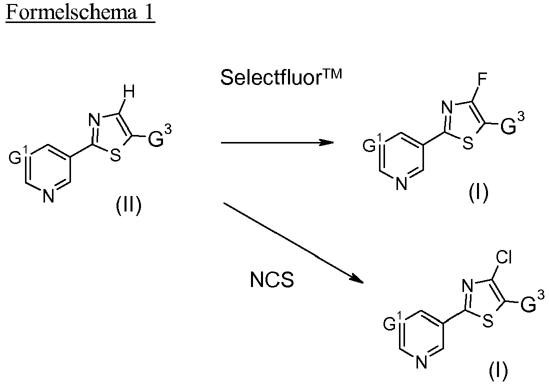 Verbindungen des Typs (II) lassen sich wie in WO 2010/006713 beschrieben erhalten.

Aus ihnen werden entsprechend Formelschema 1 durch Umsetzung mit einem Fluorierungsmittel wie 1-(Chloromethyl)-4-fluoro-1,4-diazoniabicyclo[2.2.2]octanditetra-fluoroborat (Selectfluor^{™}) in einem Verdünnungsmittel wie Acetonitril die erfmdungsgemäßen Verbindungen der Formel (I) mit R¹=F oder durch Umsetzung mit einem Chlorierungsmittel wie N-Chlorsuccinimid (NCS) die erfmdungsgemäßen Verbindungen der Formel (I) mit R¹=Cl erhalten.

Eine Fluorierung dieser Art ist beispielsweise beschrieben in US 6,231,786.

Eine Chlorierung dieser Art ist beispielsweise beschrieben in Heterocycles, 199, vol 31, Nr. 6, 1115-1127.

Eine Bromierung dieser Art ist beispielsweise beschrieben in Acta Chem. Scand. 1992, 46, 372-383. Alternativ kann die Bromverbindung durch regioselektive Palladium-katalysierte Kupplung des 4,5-Dibromthiazols mit Boronsäure oder entsprechenden metallorganischen Verbindungen hergestellt werden (Org. Lett. 2009, 11, 1475-1478).

Darüber hinaus sind 4-Cyanothiazole in der Literatur beschrieben, siehe dazu beispielsweise US 4,528,291, WO 2007/47306, US 2009/176779.

Ebenfalls beschrieben sind 4-Nitrothiazole, siehe dazu beispielsweise Journal of the American Chemical Society, 1950, vol. 72, 3188 und Bulletin de la Societe Chimique de France, 1970, 3155 - 3157.

Ebenfalls beschrieben sind 4-Bromthiazole, siehe dazu beispielsweise Heterocycles, 199, vol 31, Nr. 6, 1115-1127, Organic Letters, 2002 , vol. 4, Nr. 8 ,1363 - 1366 und New Journal of Chemistry, 2005 , vol. 29, Nr. 3, 439 - 446.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..
Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.
Aus der Klasse der Bivalva z.B. Dreissena spp..
Aus der Ordnung der Chilopoda z.B. Geophilus spp., Scutigera spp..
Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchiclius obtectus, Bruchus spp., Ceuthorhynchus spp., Cleonus menclicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..
Aus der Ordnung der Collembola z.B. Onychiurus armatus.
Aus der Ordnung der Dermaptera z.B. Forficula auricularia.
Aus der Ordnung der Diplopoda z.B. Blaniulus guttulatus.
Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp., Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.
Aus der Klasse der Gastropoda z.B. Arion spp., Biomphalaria spp., Bulinus spp., Deroceras spp., Galba spp., Lymnaea spp., Oncomelania spp., Succinea spp..
Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.
   Weiterhin lassen sich Protozoen, wie Eimeria, bekämpfen.
Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.
Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboriclia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandi-cola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.
Aus der Ordnung der Hymenoptera z.B. Diprion spp., Hoplocampa spp., Lasius spp., Monomorium pharaonis, Vespa spp..
Aus der Ordnung der Isopoda z.B. Armadillidium vulgare, Oniscus asellus, Porcellio scaber.
Aus der Ordnung der Isoptera z.B. Reticulitermes spp., Odontotermes spp..
Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..
Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.
Aus der Ordnung der Siphonaptera z.B. Ceratophyllus spp., Xenopsylla cheopis.
Aus der Ordnung der Symphyla z.B. Scutigerella immaculata.
Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..
Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die erfindungsgemäßen Verbindungen können in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP-POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Die erfindungsgemäßen Wirkstoffe können als solche oder in ihren Formulierungen auch in Mischung mit einem oder mehreren geeigneten Fungiziden, Bakteriziden, Akariziden, Nematiziden, Insektiziden, Mikrobiziden, Düngemittel, Lockstoffen, Sterilantien, Synergisten, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Des weiteren können solche Kombinationen das Pflanzenwachstum verbessern, die Toleranz gegenüber hohen oder niedrigen Temperaturen, gegen Trockenheit oder gegen Wasser- bzw. Bodensalzgehalt erhöhen, die Blühleistung steigern, die Ernte erleichtern und Ernteerträge steigern, die Reife beschleunigen, die Qualität und/oder den Ernährungswert der Ernteprodukte steigern, die Lagerfähigkeit verlängern und/oder die Bearbeitbarkeit der Ernteprodukte verbessern. In der Regel erhält man durch Kombination der erfmdungsgemäßen Wirkstoffe und Mischpartnern synergistische Effekte, d.h. die Wirksamkeit der jeweiligen Mischung ist größer als die Wirksamkeit der Einzelkomponenten. Generell können die Kombinationen sowohl als Saatgutanwendungen als auch in Vor-, Tank- oder Fertigmischungen verwendet werden.

Besonders günstige Mischungspartner sind z.B. die folgenden:

### Insektizide / Akarizide / Nematizide:

Die hier mit ihrem "common name" genannten Wirkstoffe sind bekannt und beispielsweise im Pestizidhandbuch ("The Pesticide Manual" 14th Ed., British Crop Protection Council 2006) beschrieben oder im Internet recherchierbar (z.B. http://www.alanwood.net/pesticides).
(1) Acetylcholinesterase (AChE) Inhibitoren, wie beispielsweise
   Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thioclicarb, Thiofanox, Triazamate, Trimethacarb, XMC und Xylylcarb; oder
   Organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise
   Cyclodien-organochlorine, z.B. Chlordane und Endosulfan; oder
   Phenylpyrazole (Fiprole), z.B. Ethiprole und Fipronil.
(3) Natrium-Kanal-Modulatoren / Spannungsabhängige Natrium-Kanal-Blocker, wie beispielsweise
   Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpro-pathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin; oder
   DDT; oder Methoxychlor.
(4) Nikotinerge Acetylcholin-Rezeptor (nAChR) Agonisten, wie beispielsweise
   Neonikotinoide, z.B. Acetamiprid, Clothianidin, Dinotefuran, Imidacloprid, Nitenpyram, Thiacloprid und Thiamethoxam; oder
   Nikotin.
(5) Nikotinerge Acetylcholin-Rezeptor (nAChR) allosterische Aktivatoren, wie beispielsweise
   Spinosine, z.B. Spinetoram und Spinosad.
(6) Chlorid-Kanal-Aktivatoren, wie beispielsweise
   Avermectine/Milbemycine, z.B. Abamectin, Emamectin-benzoat, Lepimectin und Milbemectin.
(7) Juvenilhormon-Imitatoren, wie beispielsweise
   Juvenilhormon-Analoge, z.B. Hydroprene, Kinoprene und Methoprene; oder
   Fenoxycarb; oder Pyriproxyfen.
(8) Wirkstoffe mit unbekannten oder nicht spezifischen Wirkmechanismen, wie beispielsweise
   Begasungsmittel, z.B. Methylbromid und andere Alkylhalogenide; oder
   Chloropicrin; oder Sulfurylfluorid; oder Borax; oder Brechweinstein.
(9) Selektive Fraßhemmer, z.B. Pymetrozine; oder Flonicamid.
(10) Milbenwachstumsinhibitoren, z.B. Clofentezine, Hexythiazox und Diflovidazin; oder Etoxazole.
(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron; oder
   Organozinnverbindungen, z.B. Azocyclotin, Cyhexatin und Fenbutatin-oxid; oder
   Propargite; oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsstörende Wirkstoffe, Dipteran, wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon; oder Acequinocyl; oder Fluacrypyrim.
(21) Komplex-1-Elektronentransportinhibitoren, beispielsweise
   METI-Akarizide, z.B. Fenazaquin, Fenpyroximate, Pyrimidifen, Pyridaben, Tebufenpyrad und Tolfenpyrad; oder
   Rotenone (Derris).
(22) Spannungsabhängige Natriumkanal-Blocker, z.B. Indoxacarb; oder Metaflumizone.
(23) Inhibitoren der Acetyl-CoA-Carboxylase, wie beispielsweise
   Tetron- und Tetramsäurederivate, z.B. Spirodiclofen, Spiromesifen und Spirotetramat.
(24) Komplex-IV-Elektronentransportinhibitoren, wie beispielsweise
   Phosphine, z.B. Aluminiumphosphid, Calciumphosphid, Phosphin und Zinkphosphid; oder
   Cyanid.
(25) Komplex-II-Elektronentransportinhibitoren, wie beispielsweise Cyenopyrafen.
(28) Ryanodinrezeptor-Effektoren, wie beispielsweise
Diamide, z.B. Chlorantraniliprole und Flubendiamide.

Weitere Wirkstoffe mit unbekanntem Wirkmechanismus, wie beispielsweise Amidoflumet, Azadirachtin, Benclothiaz, Benzoximate, Bifenazate, Bromopropylate, Chinomethionat, Cryolite, Cyantraniliprole (Cyazypyr), Cyflumetofen, Dicofol, Diflovidazin, Fluensulfone, Flufenerim, Flufiprole, Fluopyram, Fufenozide, Imidadothiz, Iprodione, Pyridalyl, Pyrifluquinazon und Iodmethan; desweiteren Präparate auf Basis von Bacillus firmus (I-1582, BioNeem, Votivo) sowie folgende bekannte wirksame Verbindungen:
3-Brom-N- {2-brom-4-chlor-6-[(1-cyclopropylethyl)carbamoyl]phenyl} -1-(3-chlorpyridin-2-yl)-1H-pyrazol-5-carboxamid (bekannt aus WO2005/077934), 4-{[(6-Brompyrid-3-yl)methyl](2-fluorethyl)amino}furan-2(5H)-on (bekannt aus WO2007/115644), 4-{[(6-Fluorpyrid-3-yl)methyl](2,2-difluorethyl)amino}furan-2(5H)-on (bekannt aus WO2007/115644), 4-{[(2-Chlor-1,3-thiazol-5-yl)methyl](2-fluorethyl)amino}furan-2(5H)-on (bekannt aus WO2007/115644), 4-{[(6-Chlorpyrid-3-yl)methyl](2-fluorethyl)amino}furan-2(5H)-on (bekannt aus WO2007/115644), 4-{[(6-Chlorpyrid-3-yl)methyl](2,2-difluorethyl)amino}furan-2(5H)-o n (bekannt aus s WO2007/115644), 4-{[(6-Chlor-5-fluorpyrid-3-yl)methyl](methyl)amino}furan-2(5H)-on (bekannt aus WO2007/115643), 4-{[(5,6-Dichlorpyrid-3-yl)methyl](2-fluorethyl)amino}furan-2(5H)-on (bekannt aus WO2007/115646), 4-{[(6-Chlor-5-fluorpyrid-3-yl)methyl](cyclopropyl)amino}furan-2(5H)-o n (bekannt aus WO2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), {[1-(6-Chlorpyridin-3-yl)ethyl](methyl)oxido-λ⁴-sulfanyliden}cyanamid (bekannt aus WO2007/149134) und seine Diastereomere {[(1R)-1-(6-Chlorpyridin-3-yl)ethyl](methyl)oxido-λ⁴-sulfanyliden}cyanamid (A) und {[(1S)-1-(6-Chlorpyridin-3-yl)ethyl](methyl)oxido-λ⁴-sulfanyliden}cyanamid (B) (ebenfalls bekannt aus WO2007/149134) sowie Sulfoxaflor (ebenfalls bekannt aus WO2007/149134) und seine Diastereomere [(R)-Methyl(oxido){(1R)-1-[6-(trifluormethyl)pyridin-3-yl]ethyl}-λ⁴-sulfanyliden]cyanamid (A1) und [(S)-Methyl(oxido) {(1S)-1-[6-(trifluormethyl)pyridin-3-yl]ethyl}-λ⁴-sulfanyliden]cyanamid (A2), bezeichnet als Diastereomerengruppe A (bekannt aus WO 2010/074747, WO 2010/074751), [(R)-Methyl(oxido) {(1S)-1-[6-(trifluormethyl)pyridin-3-yl]ethyl}-λ⁴-sulfanyliden]cyanamid (B1) und [(S)-Methyl(oxido){(1R)-1-[6-(trifluormethyl)pyridin-3-yl]ethyl}-λ⁴-sulfanyliden]cyanamid (B2), bezeichnet als Diastereomerengruppe B (ebenfalls bekannt aus WO 2010/074747, WO 2010/074751) und 11-(4-Chlor-2,6-dimethylphenyl)-12-hydroxy-1,4-dioxa-9-azadispiro[4.2.4.2]tetradec-11-en-10-on (bekannt aus WO2006/089633), 3-(4'-Fluor-2,4-dimethylbiphenyl-3-yl)-4-hydroxy-8-oxa-1-azaspiro[4.5]dec-3-en-2-o n (bekannt aus s WO2008/067911), 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), [(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat (bekannt aus WO2008/066153), 2-Cyan-3-(difluormethoxy)-N,N-dimethylbenzolsulfonamid (bekannt ausWO2006/056433), 2-Cyan-3-(difluormethoxy)-N-methylbenzolsulfonamid (bekannt aus WO2006/100288), 2-Cyan-3-(difluormethoxy)-N-ethylbenzolsulfonamid (bekannt aus WO2005/035486), 4-(Difluormethoxy)-N-ethyl-N-methyl-1,2-benzothiazol-3-amin-1,1-dioxid (bekannt aus WO2007/057407), N-[1-(2,3-Dimethylphenyl)-2-(3,5-dimethylphenyl)ethyl]-4,5-dihydro-1,3-thiazol-2-amin (bekannt aus WO2008/104503), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), (2,2,3,3,4,4,5,5-Octafluorpentyl)(3,3,3-trifluorpropyl)malononitril (bekannt aus WO2005/063094), (2,2,3,3,4,4,5,5-Octafluorpentyl)(3,3,4,4,4-pentafluorbutyl)malononitril (bekannt aus WO2005/063094), 8-[2-(Cyclopropylmethoxy)-4-(trifluormethyl)phenoxy]-3-[6-(trifluormethyl)pyridazin-3-yl]-3-azabicyclo[3.2.1]octan (bekannt aus WO2007/040280), 2-Ethyl-7-methoxy-3-methyl-6-[(2,2,3,3-tetrafluor-2,3-dihydro-1,4-benzodioxin-6-yl)oxy]chinolin-4-yl-methylcarbonat (bekannt aus JP2008/110953), 2-Ethyl-7-methoxy-3-methyl-6-[(2,2,3,3-tetrafluor-2,3-dihydro-1,4-benzodioxin-6-yl)oxy]chinolin-4-ylacetat (bekannt aus JP2008/110953), PF1364 (CAS-Reg.Nr. 1204776-60-2) (bekannt aus JP2010/018586), 5-[5-(3,5-Dichlorphenyl)-5-(trifluormethyl)-4,5-dihydro-1,2-oxazol-3-yl]-2-(1H-1,2,4-triazol-1-yl)benzonitril (bekannt aus WO2007/075459), 5-[5-(2-Chlorpyridin-4-yl)-5-(trifluormethyl)-4,5-dihydro-1,2-oxazol-3-yl]-2-(1H-1,2,4-triazol-1-yl)benzonitril (bekannt aus WO2007/075459), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (bekannt aus WO2005/08521 6), 4-{[(6-Chlorpyridin-3-yl)methyl](cyclopropyl)amino}-1,3-oxazol-2(5H)-on, 4-{[(6-Chlorpyridin-3-yl)methyl](2,2-difluorethyl)amino}-1,3-oxazol-2(5H)-on, 4-{[(6-Chlorpyridin-3-yl)methyl](ethyl)amino}-1,3-oxazol-2(5H)-on, 4-{[(6-Chlorpyridin-3-yl)methyl](methyl)amino}-1,3-oxazol-2(5H)-on (alle bekannt aus WO2010/005692), NNI-0711 (bekannt aus WO2002096882), 1-Acetyl-N-[4-(1,1,1,3,3,3-hexafluor-2-methoxypropan-2-yl)-3-isobutylphenyl]-N-isobutyryl-3,5-dimethyl-1H-pyrazol-4-carboxamid (bekannt aus WO2002096882), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus s WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-1,2-diethylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), (5RS,7RS;5RS,7SR)-1-(6-Chlor-3-pyridylmethyl)-1,2,3,5,6,7-hexahydro-7-methyl-8-nitro-5-propoxyimidazo[1,2-a]pyridin (bekannt aus WO2007/101369), 2-{6-[2-(5-Fluorpyridin-3-yl)-1,3-thiazol-5-yl]pyridin-2-yl}pyrimidin (bekannt aus WO2010/006713), 2-{6-[2-(Pyridin-3-yl)-1,3-thiazol-5-yl]pyridin-2-yl}pyrimidin (bekannt aus WO2010/006713).

### Fungizide

(1) Inhibitoren der Ergosterol-Biosynthese, wie beispielsweise Aldimorph, Azaconazol, Bitertanol, Bromuconazol, Cyproconazol, Diclobutrazol, Difenoconazol, Diniconazol, Diniconazol-M, Dodemorph, Dodemorph Acetat, Epoxiconazol, Etaconazol, Fenarimol, Fenbuconazol, Fenhexamid, Fenpropiclin, Fenpropimorph, Fluquinconazol, Flurprimidol, Flusilazol, Flutriafol, Furconazol, Furconazol-Cis, Hexaconazol, Imazalil, Imazalil Sulfat, Imibenconazol, Ipconazol, Metconazol, Myclobutanil, Naftifin, Nuarimol, Oxpoconazol, Paclobutrazol, Pefurazoat, Penconazol, Piperalin, Prochloraz, Propiconazol, Prothioconazol, Pyributicarb, Pyrifenox, Quinconazol, Simeconazol, Spiroxamin, Tebuconazol, Terbinafin, Tetraconazol, Triadimefon, Triadimenol, Tridemorph, Triflumizol, Triforin, Triticonazol, Uniconazol, Uniconazol-p, Viniconazol, Voriconazol, 1-(4-Chlorphenyl)-2-(1H-1,2,4-triazol-1-yl)cycloheptanol, Methyl-1-(2,2-dimethyl-2,3-dihydro-1H-inden-1-yl)-1H-imidazol-5-carboxylat, N'-{5-(Difluormethyl)-2-methyl-4-[3-(trimethylsilyl)propoxy]phenyl} -N-ethyl-N-methylimidoformamid, N-Ethyl-N-methyl-N'- {2-methyl-5-(trifluormethyl)-4-[3-(trimethylsilyl)propoxy]phenyl}imidoformamid und O-[1-(4-Methoxyphenoxy)-3,3-dimethylbutan-2-yl]-1H-imidazol-1-carbothioat.
(2) Inhibitoren der Respiration (Atmungsketten-Inhibitoren), wie beispielsweise Bixafen, Boscalid, Carboxin, Diflumetorim, Fenfuram, Fluopyram, Flutolanil, Fluxapyroxad, Furametpyr, Furmecyclox, Isopyrazam Mischung des syn-epimeren Razemates 1RS,4SR,9RS und des anti-empimeren Razemates 1RS,4SR,9SR, Isopyrazam (anti-epimeres Razemat), Isopyrazam (anti-epimeres Enantiomer 1R,4S,9S), Isopyrazam (anti-epimeres Enantiomer 1S,4R,9R), Isopyrazam (syn-epimeres Razemat 1RS,4SR,9RS), Isopyrazam (syn-epimeres Enantiomer 1R,4S,9R), Isopyrazam (syn-epimeres Enantiomer 1S,4R,9S), Mepronil, Oxycarboxin, Penflufen, Penthiopyrad, Sedaxane, Thifluzamid, 1-Methyl-N-[2-(1,1,2,2-tetrafluorethoxy)phenyl]-3-(trifluormethyl)-1H-pyrazol-4-carboxamid, 3-(Difluormethyl)-1-methyl-N-[2-(1,1,2,2-tetrafluorethoxy)phenyl]-1H-pyrazol-4-carboxamid, 3-(Difluormethyl)-N-[4-fluor-2-(1,1,2,3,3,3-hexafluorpropoxy)phenyl]-1-methyl-1H-pyrazol-4-carboxamid und N-[1-(2,4-Dichlorphenyl)-1-methoxypropan-2-yl]-3-(difluormethyl)-1-methyl-1 H-pyrazol-4-carboxamid.
(3) Inhibitoren der Respiration (Atmungsketten-Inhibitoren) am Komplex III der Atumungskette, wie beispielsweise Ametoctradin, Amisulbrom, Azoxystrobin, Cyazofamid, Dimoxystrobin, Enestroburin, Famoxadon, Fenamidon, Fluoxastrobin, Kresoxim-Methyl, Metominostrobin, Orysastrobin, Picoxystrobin, Pyraclostrobin, Pyrametostrobin, Pyraoxystrobin, Pyribencarb, Trifloxystrobin, (2E)-2-(2- {[6-(3-Chlor-2-methylphenoxy)-5-fluorpyrimidin-4-yl]oxy}phenyl)-2-(methoxyimino)-N-methylethanamid, (2E)-2-(Methoxyimino)-N-methyl-2-(2-{[({(1E)-1-[3-(trifluormethyl)phenyl]ethyliden}amino)oxy]methyl}phenyl)ethanamid, (2E)-2-(Methoxyimino)-N-methyl-2- {2-[(E)-( {1-[3-(trifluormethyl)phenyl]ethoxy}imino)methyl]phenyl} ethanamid, (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid, (2E)-2-{2-[({[(2E,3E)-4-(2,6-Dichlorphenyl)but-3-en-2-yliden]amino}oxy)methyl]phenyl}-2-(methoxyimino)-N-methylethanamid, 2-Chlor-N-(1,1,3-trimethyl-2,3-dihydro-1H-inden-4-yl)pyridin-3-carboxamid, 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, Methyl-(2E)-2- {2-[({cyclopropyl[(4-methoxyphenyl)imino]methyl}sulfanyl)methyl]phenyl}-3-methoxyprop-2-enoat, N-(3-Ethyl-3,5,5-trimethylcyclohexyl)-3-(formylamino)-2-hydroxybenzamid, 2- {2-[(2,5-Dimethylphenoxy)methyl]phenyl}-2-methoxy-N-methylacetamid und (2R)-2- {2-[(2,5-Dimethylphenoxy)methyl]phenyl} -2-methoxy-N-methylacetamid.
(4) Inhibitoren der Mitose und Zellteilung, wie beispielsweise Benomyl, Carbendazim, Chlorfenazol, Diethofencarb, Ethaboxam, Fluopicolid, Fuberidazol, Pencycuron, Thiabendazol, Thiophanat-Methyl, T h i ophanat, Zoxamid, 5-Chlor-7-(4-methylpiperidin-1-yl)-6-(2,4,6-trifluorphenyl)[1,2,4]triazolo[1,5-a]pyrimidin und 3-Chlor-5-(6-chlorpyridin-3-yl)-6-methyl-4-(2,4,6-trifluorphenyl)pyridazin.
(5) Verbindungen mit Multisite-Aktivität, wie beispielsweise Bordeauxmischung, Captafol, Captan, Chlorothalonil, Kupferzubereitungen wie Kupferhydroxid, Kupfernaphthenat, Kupferoxid, Kupferoxychlorid, Kupfersulfat, Dichlofluanid, Dithianon, Dodine, Dodine freie Base, Ferbam, Fluorofolpet, Folpet, Guazatin, Guazatinacetat, Iminoctadin, Iminoctadinalbesilat, Iminoctadintriacetat, Mankupfer, Mancozeb, Maneb, Metiram, Zinkmetiram, Kupfer-Oxin, Propamidin, Propineb, Schwefel und Schwefelzubereitungen wie beispielsweise Calciumpolysulfid, Thiram, Tolylfluanid, Zineb und Ziram.
(6) Resistenzinduktoren, wie beispielsweise Acibenzolar-S-Methyl, Isotianil, Probenazol und Tiadinil.
(7) Inhibitoren der Aminosäure- und Protein-Biosynthese, wie beispielsweise Andoprim, Blasticidin-S, Cyprodinil, Kasugamycin, Kasugamycin Hydrochlorid Hydrat, Mepanipyrim und Pyrimethanil.
(8) Inhibitoren der ATP Produktion, wie beispielsweise Fentin Acetat, Fentin Chlorid, Fentin Hydroxid und Silthiofam.
(9) Inhibitoren der Zellwandsynthese, wie beispielsweise Benthiavalicarb, Dimethomorph, Flumorph, Iprovalicarb, Mandipropamid, Polyoxins, Polyoxorim, Validamycin A und Valifenalat.
(10) Inhibitoren der Lipid- und Membran-Synthese, wie beispielsweise Biphenyl, Chloroneb, Dicloran, Edifenphos, Etridiazol, Iodocarb, Iprobenfos, Isoprothiolan, Propamocarb, Propamocarb Hydrochlorid, Prothiocarb, Pyrazophos, Quintozen, Tecnazene und Tolclofos-Methyl.
(11) Inhibitoren der Melanin-Biosynthese, wie beispielsweise Carpropamid, Diclocymet, Fenoxanil, Fthalid, Pyroquilon und Tricyclazol.
(12) Inhibitoren der Nukleinsäuresynthese, wie beispielsweise Benalaxyl, Benalaxyl-M (Kiralaxyl), Bupirimat, Clozylacon, Dimethirimol, Ethirimol, Furalaxyl, Hymexazol, Metalaxyl, Metalaxyl-M (Mefenoxam), Ofurace, Oxadixyl, Oxolinsäure.
(13) Inhibitoren der Signaltransduktion, wie beispielsweise Chlozolinat, Fenpiclonil, Fluclioxonil, Iprodion, Procymidon, Quinoxyfen und Vinclozolin.
(14) Entkoppler, wie beispielsweise Binapacryl, Dinocap, Ferimzon, Fluazinam und Meptyldinocap.
(15) Weitere Verbindungen, wie beispielsweise Benthiazol, Bethoxazin, Capsimycin, Carvon, Chinomethionat, Chlazafenon, Cufraneb, Cyflufenamid, Cymoxanil, Cyprosulfamide, Dazomet, Debacarb, Dichlorophen, Diclomezin, Difenzoquat, Difenzoquat Methylsulphat, Diphenylamin, Ecomat, Fenpyrazamin, Flumetover, Fluoromid, Flusulfamid, Flutianil, Fosetyl-Aluminium, Fosetyl-Calcium, Fosetyl-Natrium, Hexachlorbenzol, Irumamycin, Methasulfocarb, Methylisothiocyanat, Metrafenon, Mildiomycin, Natamycin, Nickel Dimethyldithiocarbamat, Nitrothal-Isopropyl, Octhilinone, Oxamocarb, Oxyfenthiin, Pentachlorphenol und dessen Salze, Phenothrin, Phosphorsäure und deren Salze, Propamocarb-Fosetylat, Propanosin-Natrium, Proquinazid, Pyrrolnitrin, Tebufloquin, Tecloftalam, Tolnifanid, Triazoxid, Trichlamid, Zarilamid, 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-Methoxyphenoxy)-3,3-dimethylbutan-2-yl-1H-imidazol-1-carboxylat, 2,3,5,6-Tetrachlor-4-(methylsulfonyl)pyridin, 2,3-Dibutyl-6-chlorthieno[2,3-d]pyrimidin-4(3H)-on, 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, 2-Butoxy-6-iod-3-propyl-4H-chromen-4-on, 2-Chlor-5-[2-chlor-1-(2,6-difluor-4-methoxyphenyl)-4-methyl-1H-imidazol-5-yl]pyridin, 2-Phenylphenol und dessen Salze, 3,4,5-Trichlorpyridin-2,6-dicarbonitril, 3-[5-(4-Chlorphenyl)-2,3-dimethyl-1,2-oxazolidin-3-yl]pyridin, 3-Chlor-5-(4-chlorphenyl)-4-(2,6-difluorphenyl)-6-methylpyridazin, 4-(4-Chlorphenyl)-5-(2,6-difluorphenyl)-3,6-dimethylpyridazin, 5-Amino-1,3,4-thiadiazol-2-thiol, 5-Chlor-N'-phenyl-N'-(prop-2-in-1-yl)thiophen-2-sulfonohydrazid, 5-Methyl-6-octyl[1,2,4]triazolo[1,5-a]pyrimidin-7-amin, Ethyl-(2Z)-3-amino-2-cyan-3-phenylprop-2-enoat, N-(4-Chlorbenzyl)-3-[3-methoxy-4-(prop-2-in-1-yloxy)phenyl]propanamid, N-[(4-Chlorphenyl)(cyan)methyl]-3-[3-methoxy-4-(prop-2-in-1-yloxy)phenyl]propanamid, N-[(5-Brom-3-chlorpyridin-2-yl)methyl]-2,4-dichlorpyridin-3-carboxamid, N-[1-(5-Brom-3-chlorpyridin-2-yl)ethyl]-2,4-dichlorpyridin-3-c arboxamid, N-[1-(5-Brom-3-chlorpyridin-2-yl)ethyl]-2-fluor-4-iodpyridin-3-carboxamid, N-{(E)-[(Cyclopropylmethoxy)imino][6-(difluormethoxy)-2,3-difluorphenyl]methyl}-2-phenylacetamid, N-{(Z)-[(Cyclopropylmethoxy)imino][6-(difluormethoxy)-2,3-difluorphenyl]methyl}-2-phenylacetamid, N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazol-4-carboxamid, N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazol-4-carboxamid, N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazol-4-carboxamid, Penty1-{6-[({[(1-methyl-1H-tetrazol-5-yl)(phenyl)methyliden]amino}oxy)methyl]pyridin-2-yl}carbamat, Phenazin-1-carbonsäure, Chinolin-8-ol und Chinolin-8-olsulfat(2:1).
(16) Weitere Verbindungen, wie beispielsweise 1-Methyl-3-(trifluormethyl)-N-[2'-(trifluormethyl)biphenyl-2-yl]-1H-pyrazol-4-carboxamid,N-(4'-Chlorbiphenyl-2-yl)-3-(difluormethyl)-1-methyl-1H-pyrazol-4-carboxamid, N-(2',4'-Dichlorbiphenyl-2-yl)-3-(difluormethyl)-1-methyl-1H-pyrazol-4-carboxamid, 3-(Difluormethyl)-1-methyl-N-[4'-(trifluormethyl)biphenyl-2-yl]-1H-pyrazol-4-carboxamid, N-(2',5'-Difluorbiphenyl-2-yl)-1-methyl-3-(trifluormethyl)-1H-pyrazol-4-carboxamid, 3-(Difluormethyl)-1-methyl-N-[4'-(prop-1-in-1-yl)biphenyl-2-yl]-1H-pyrazol-4-carboxamid, 5-Fluor-1,3-dimethyl-N-[4'-(prop-1-in-1-yl)biphenyl-2-yl]-1H-pyrazol-4-carboxamid, 2-Chlor-N-[4'-(prop-1-in-1-yl)biphenyl-2-yl]pyridin-3-carboxamid, 3-(Difluormethyl)-N-[4'-(3,3-dimethylbut-1-in-1-yl)biphenyl-2-yl] -1-methyl-1 H-pyrazol-4-carboxamid, N-[4'-(3,3-Dimethylbut-1-in-1-yl)biphenyl-2-yl]-5-fluor-1,3-dimethyl-1H-pyrazol-4-carboxamid, 3-(Difluormethyl)-N-(4'-ethinylbiphenyl-2-yl)-1-methyl-1H-pyrazol-4-carboxamid, N-(4'-Ethinylbiphenyl-2-yl)-5-fluor-1,3-dimethyl-1H-pyrazol-4-carboxamid, 2-Chlor-N-(4'-ethinylbiphenyl-2-yl)pyridin-3-carboxamid, 2-Chlor-N-[4'-(3,3-dimethylbut-1-in-1 -yl)biphenyl-2-yl]pyridin-3-carboxamid, 4-(Difluormethyl)-2-methyl-N-[4'-(trifluormethyl)biphenyl-2-yl]-1,3-thiazol-5-carboxamid, 5-Fluor-N-[4'-(3-hydroxy-3-methylbut-1-in-1-yl)biphenyl-2-yl]-1,3-dimethyl-1H-pyrazol-4-carboxamid, 2-Chlor-N-[4'-(3-hydroxy-3-methylbut-1-in-1-yl)biphenyl-2-yl]pyridin-3-carboxamid, 3-(Difluormethyl)-N-[4'-(3-methoxy-3-methylbut-1-in-1-yl)biphenyl-2-yl]-1-methyl-1H-pyrazol-4-carboxamid, 5-Fluor-N-[4'-(3-methoxy-3-methylbut-1-in-1-yl)biphenyl-2-yl]-1,3-dimethyl-1H-pyrazol-4-carboxamid, 2-Chlor-N-[4'-(3-methoxy-3-methylbut-1-in-1-yl)biphenyl-2-yl]pyridin-3-carboxamid, ( 5-Brom-2-methoxy-4-methylpyridin-3-yl)(2,3,4-trimethoxy-6-methylphenyl)methanon und N-[2-(4-{[3-(4-Chlorphenyl)prop-2-in-1-yl]oxy}-3-methoxyphenyl)ethyl]-N2-(methylsulfonyl)valinamid.

Alle genannten Mischpartner der Klassen (1) bis (16) können, wenn sie auf Grund ihrer funktionellen Gruppen dazu imstande sind, gegebenenfalls mit geeigneten Basen oder Säuren Salze bilden.

Jeder zusätzliche Wirkstoff kann in einem Gewichtsverhältnis von 100:1 bis 1:100, bevorzugt von 5 : 1 bis 1 : 5, mit den erfindungsgemäßen Wirkstoffen gemischt werden.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfmdungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfmdungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfmdungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfmdungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und Cry-IF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylhamstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizidtolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp. (Ctenocephalides canis, Ctenocephalides felis), Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfred-dugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Pemphigus spp., Phylloera vastatrix, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Herstellungsbeispiele

### Beispiel 1

### 2-[6-(4-Fluoro-2-pyridin-3-yl-thiazol-5-yl)-pyridin-2-yl]-pyrimidin

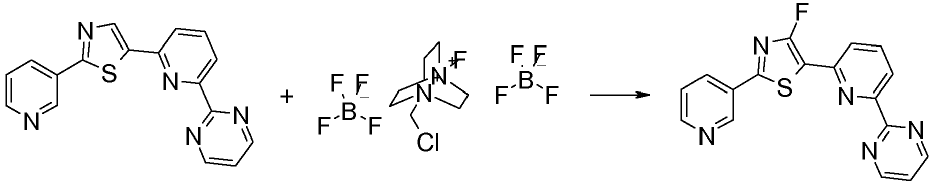

0,5 g (1,57 mmol) 2-[6-(2-Pyridin-3-yl-thiazol-5-yl)-pyridin-2-yl]-pyrimidin wurden in Acetonitril gelöst, mit 1,16 g (3,15 mmol) 1-(Chloromethyl)-4-fluoro-1,4-cliazoniabi-cyclo[2.2.2]octanditetrafluoroborat (Selectfluor ™) sowie 1g (7,04 mmol) Natriumsulfat versetzt, über Nacht bei Rückfluß erhitzt und nach Abkühlen über Celite filtriert. Das Filtrat wurde eingeengt und durch Chromatographie (Cyclohexan/Ethylacetat, dann Ethylacetat/Methanol) gereinigt.
Ausbeute: 62,0 mg (11% d. Th.);
HPLC-MS: logP(HCOOH) = 2,15 ; Masse (m/z): 336,1 (M+H)⁺; ¹H-NMR (D6-DMSO): 7,60 (m, 2H), 8,00 (d, 1H), 8,15 (t, 1H), 8,35 (d, 1H), 8,40 (d, 1H), 8,75 (d, 1H), 9,05 (m, 2H), 9,20 ppm (m, 1H).

### Beispiel 2

### 2-{6-[4-Chlor-2-(pyridin-3-yl)-1,3-thiazol-5-yl]pyridin-2-yl}pyrimidin und 5-Chlor-2-{6-[4-chlor-2-(pyridin-3-yl)-1,3-thiazol-5-yl]pyridin-2-yl}pyrimidin

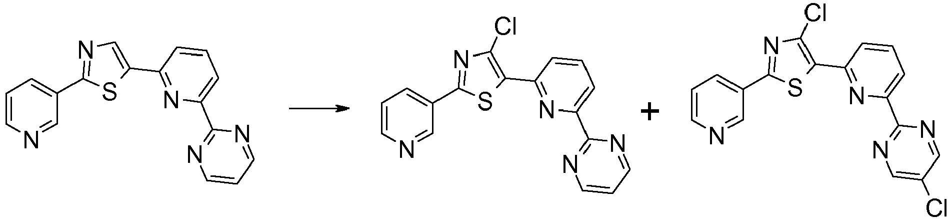

325 mg (993 µmol) 2-[6-(2-Pyridin-3-yl-thiazol-5-yl)-pyridin-2-yl]-pyrimidin wurden in 6 ml Dimethylformamid gelöst, mit 146 mg (1,09 mmol) N-Chlorsuccinimid versetzt und für 6 Stunden auf 80 °C erhitzt. Nach erneuter Zugabe von N-Chlorsuccinimid (1 äq.) wurde für weitere 14 Stunden bei 80 °C gerührt. Das Reaktionsgemisch wurde auf Wasser gegeben und mehrmals mit Ethylacetat extrahiert. Die vereinigten organischen Phasen wurden über Magnesiumsulfat getrocknet, filtriert und eingeengt. Das Rohprodukt wurde chromatographisch aufgereinigt (Cyclohexan/Ethylacetat).

### 2-{6-[4-Chlor-2-(pyridin-3-yl)-1,3-thiazol-5-yl]pyridin-2-yl}pyrimidin

Ausbeute: 52,2 mg (15% d. Th.);
HPLC-MS: logP (HCOOH) = 2,27 ; Masse (m/z): 352,0 (M+H)⁺; ¹H-NMR (D6-DMSO): 7,38 (t, 1H), 7,42 (m, 1H), 8,02 (t, 1H), 8,31 (m, 1H), 8,41 (d, 1H), 8,47 (d, 1H), 8,70 (m, 1H), 8,97 (d, 2H), 9,24 ppm (m, 1H).

### 5-Chlor-2-{6-[4-chlor-2-(pyridin-3-yl)-1,3-thiazol-5-yl]pyridin-2-yl}pyrimidin

Ausbeute: 15 mg (4% d. Th.);
HPLC-MS: logP (HCOOH) = 3,27 ; Masse (m/z): 385,9; 387,9 (M+H)⁺; ¹H-NMR (D6-DMSO): 7,44 (m, 1H), 8,02 (t, 1H), 8,33 (m, 1H), 8,41 (d, 1H), 8,43 (d, 1H), 8,71 (m, 1H), 8,91 (s, 2H), 9,24 ppm (m, 1H).

### 1) Methodenbeschreibung zur Bestimmung der logP Werte (Ameisensäure Methode)

Die Bestimmung der logP-Werte erfolgte gemäß EEC-Directive 79/831 Annex V.A8 durch HPLC (High Performance Liquid Chromato-graphy) an einer Phasenumkehrsäule (C 18). Temperatur: 55°C.

Eluenten für die Bestimmung im sauren Bereich (pH 3,4):
Eluenat A: Acetonitril + 1 ml Ameisensäure/Liter. Eluent B: Wasser + 0,9 ml Ameisensäure/Liter.
Gradient: von 10% Eluent A / 90% Eluent B bis 95% Eluent A / 5% Eluent B in 4,25 min.

Die Eichung erfolgte mit unverzweigten Alkan-2-onen (mit 3 bis 16 Kohlen-stoff-atomen), deren logP-Werte bekannt sind (Bestimmung der logP-Werte anhand der Retentionszeiten durch lineare Interpolation zwischen zwei aufeinander folgenden Alkanonen). Die lambda-max-Werte wurden an Hand der UV-Spektren von 200 nm bis 400 nm in den Maxima der chromatographischen Signale ermittelt.

### Biologische Beispiele

### Myzus-Test (Spritzbehandlung)

| | |
|---|---|
| Lösungsmittel: | 78 GewichtsteileAceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 100 % bei einer Aufwandmenge von 500g/ha :
Bsp. Nr. 1

## Patentansprüche

1. Verbindungen der Formel (I) 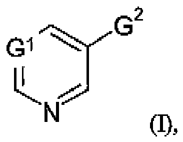 worin
G¹ für N, CH oder C-Halogen steht,
G² für 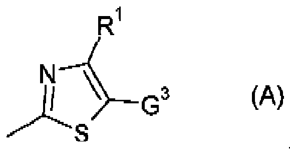 steht,
worin
R¹ für einen Rest aus der Reihe Cyano, Nitro, Fluor, Chlor, Brom und Iod steht,
G³ für einen Hoterocyclus aus der Reihe Dioxanyl, Oxazolinyl, Dihydrooxadiazinyl, Dihydrodioxazinyl und Hydroxypyridyl steht, welcher substituiert ist durch einen oder mehrere Reste aus der Reihe Halogen, Cyano, Nitro, Alkyl, Halogenalkyl, Cycloalkyl, Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl, Aminocarbonyl, Pyridyl und Pyrimidyl,
G³ auch für Heteroaryl aus der Reihe Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5,Triazinyl, Benzofuryl, Benzisofuryl, Benzothienyl, Benzisothienyl, Indolyl, Isoindalyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indalizinyl steht, welches substituiert ist durch einen Heterocyclylrest aus der Reihe Triazolinon-yl, Dihydrodioxazinyl, Dihydrooxadiazinyl, Dioxolanyl, Dioxanyl (insbesondere 1,3-Dioxan-2-yl), Piperidinonyl, Pyrrolidinonyl und Pyrazolinonyl (welcher selbst wiederum substituiert sein kann durch Alkyl und Halogenalkyl), durch Phenyl (welches selbst wiederum substituiert sein kann durch Halogen, Cyano, Nitro, Alkyl und Halogenalkyl), durch einen Heteroarylrest aus der Reihe Pyrrolyl, Pyridyl, Pyridyl-N-oxid, Pyrimidyl, Imidazolyl, Pyrazolyl, Oxazolyl, Triazolyl, Furanyl, Thienyl, Triazolyl, Oxadiazolyl, Thiadiazolyl, Pyrazinyl, Triazinyl, Tetrazinyl und Isochinolinyl (welcher selbst wiederum substituiert sein kann durch Halogen, Nitro, Alkyl, Halogenalkyl, Alkoxy, Halogenalkoxy, Alkoxyalkyl, Alkylthio, Alkylthioalkyl und Cycloalkyl) oder durch einen Heteroarylalkylrest aus der Reihe Triazolylalkyl, Pyridylalkyl, Pyrimidylalkyl und Oxadiazolylalkyl (welcher selbst wiederum substituiert sein kann durch Alkyl),
sowie Salze, Metallkomplexe und N-Oxide der Verbindungen der Formel (I).

2. Verbindungen der Formel (I) gemäß Anspruch 1, worin
G¹ für N, CH oder C-Halogen steht,
G² für 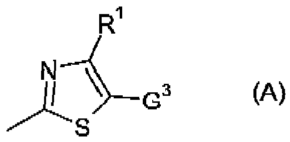 steht,
worin
R¹ für Cyano, Fluor oder Chlor steht,
G³ für einen Heterocyclus aus der Reihe 1,3-Dioxan-2-yl, Oxazolinyl, Dihydrooxadiazinyl, Dihydrodioxazinyl oder Hydroxypyridyl steht, welcher substituiert ist durch einen oder mehrere Reste aus der Reihe Halogen, Cyano, Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkylthio, C₁-C₆-Haloalkylthio, C₁-C₆-Alkylsulfinyl, C₁-C₆-Alkylsulfionyl, C₁-C₆-Haloalkylsulfinyl, C₁-C₆-Haloalkylsulfonyl, Amino, C₁-C₆-Alkylamino, Di-(C₁-C₆-alkyl)amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, G₂-C₆-Alkenyl, C₂-C₆-Alkinyl, C₃-C₆-cycloalkyl-C₁-C₆-alkyl, C₁-C₆-Alkylcarbonyl, C₁-C₆-Alkoxycarbonyl, Aminocarbonyl, Pyridyl und Pyrimidyl,
G³ auch für Heteroaryl aus der Reihe Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazoly, 1,2,5-Oxadiazolyl, 1,2,3-Thiadiazolyl, 1,2,4-Thiadiazalyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofüryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indohzinyl steht, welches jeweils substituiert ist durch einen Heteracyclylrest aus der Reihe Morpholinyl, Triazolinonyl, Dihydrodioxazinyl, Dihydrooxadiazinyl, Dioxolanyl, Dioxanyl, Piperidinonyl, Pyrrolidinonyl und Pyrazolinonyl (welcher selbst wiederum substituiert sein kann durch C₁-C₆-Alkyl und C₁-C₆-Halagenalkyl), durch Phenyl (welches selbst wiederum substituiert sein kann durch Halogen, Cyano, Nitro, C₁-C₆-Alkyl und C₁-C₆-Halogenalkyl), durch einen Heteroarylrest aus der Reihe Pyrrolyl, Pyridyl, Pyridyl-N-oxid, Pyrimidyl, Imidazolyl, Pyrazolyl, Oxazolyl, Thiazolyl, Furanyl, Thienyl, Triazolyl, Oxadiazolyl, Thiadiazolyl, Pyrazinyl, Triazinyl, Tetrazinyl und Isochinolinyl (welcher selbst wiederum substituiert sein kann durch Halogen, Nitro, C₁-C₆-Alkyl, C₁-C₆-Halogenalkyl, C₁-C₆-Alkoxy, C₁-C₆-Halogenalkoxy, C₁-C₆-Alkoxy-C₁-C₆-alkyl, C₁-C₆-Alkylthio, C₁-C₆-Alkylthio-C₁-C₆-alkyl und C₃-C₆-Cycloalkyl) oder durch einen Heteroarylalkylrest aus der Reihe Triazolyl-C₁-C₆-alkyl, Pyridyl-C₁-C₆-alkyl, Pyrimidyl-C₁-C₆-akyl und Oxadiazolyl-C₁-C₆-alkyl (welcher selbst wiederum substituiert sein kann durch C₁-C₆-Alkyl),

3. Verbindungen der Formel (I) gemäß Anspruch 1, worin
G¹ für N, CH oder C-Halogen steht,
G² für 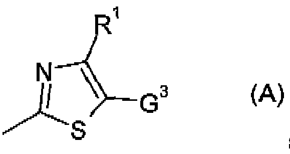 steht,
worin
R¹ für Cyano, Fluor oder Chlor steht,
G³ für einen Heterocyclus aus der Reihe 1,3-Dioxan-2-yl, Oxazolinyl, Dihydrooxadiazinyl, Dihydrodioxazinyl und Hydroxypyndyl steht, welcher substituiert ist durch einen oder mehrere Reste aus der Reihe Halogen, Cyano, Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylthio, C₁-C₄-Haloalkylthio, C₁-C₄-Alkylsulfinyl, C₁-C₄-Alkylsulfonyl, C₁-C₄-Haloalkylsulfinyl, C₁-C₄-Haloalkylsulfonyl, Amino, C₁-C₄-Alkylamino, Di-(C₁-C₄-alkyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₃-C₆-Cycloalkyl-C₁-C₄-alkyl, C₁-C₄-Alkylcarbonyl, C₁-C₄-Alkoxycarbonyl, Aminocarbonyl, Pyridyl und Pyrimidyl,
G³ auch für einen Heteroarylrest aus der Reihe Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1.2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl. Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl steht, welcher substituiert ist durch einen Heterocyclylrest aus der Reihe Morpholinyl, Triazolinonyl, Dihydrodioxazinyl, Dihydrooxadiazinyl, Dioxolanyl, Dioxanyl, Piperidinonyl, Pyrrolidinonyl und Pyrazolinonyl (welcher selbst wiederum substituiert sein kann durch C₁-C₄-Alkyl und C₁-C₄-Halogenalkyl), durch Phenyl (welches selbst wiederum substituiert sein kann durch Halogen, Cyano, Nitro, C₁-C₄-Alkyl und C₁-C₄-Halogenalkyl), durch einen Heteroarylrest aus der Reihe Pyrrolyl, Pyridyl, Pyridyl-N-oxid, Pyrimidyl, Imidazolyl, Pyrazolyl, Oxazolyl, Thiazolyl, Furanyl, Thienyl, Triazolyl, Oxadiazolyl, Thiadiazolyl, Pyrazinyl, Triazinyl, Tetrazinyl und Isochinolinyl (welcher selbst wiederum substituiert sein kann durch Halogen, Nitro, C₁-C₄-Alkyl, C₁-C₄-Halogenalkyl, C₁-C₄-Alkoxy C₁-C₄-Halogenalkoxy, C₁-C₄-Alkoxy-C₁-C₄-alkyl, C₁-C₄-Alkylthio, C₁-C₄-Alkylthio-C₁-C₄-alkyl und C₃-C₆-Cycloalkyl) oder durch einen Heteroarylalkylrest aus der Reihe Triazolyl-C₁-C₄-alkyl, Pyridyl-C₁-C₄-alkyl, Pyrimidyl-C₁-C₄-alkyl oder Oxadiazolyl-C₁-C₄-alkyl (welcher selbst wiederum substituiert sein kann durch C₁-C₄-Alkyl).

4. Verbindungen der Formel (I) gemäß Anspruch 1,
worin
G¹ für N, CH oder C-Halogen steht,
G² für 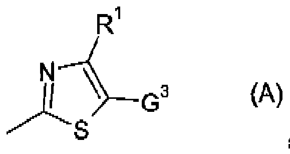 steht,
worin
R¹ für Fluor oder Chlor steht,
G³ auch für einen Heteroarylrest aus der Reihe Pyrazolyl, 1,2,4-Oxadiazolyl, Pyridyl, Pyrimidinyl, 1,3,5-Triazinyl, Triazinyl und Oxadiazolyl steht, welcher substituiert ist durch einen Heterocyclylrest aus der Reihe Morpholinyl, Triazolinonyl, Dihydrodioxazinyl, Dihydrooxadiazinyl, Piperidinonyl, Pyrrolidinonyl und Pyrazolinonyl (welcher selbst wiederum, substituiert sein kann durch C₁-C₄-Alkyl und C₁-C₄-Halogenalkyl), durch Phenyl (welches selbst wiederum substituiert sein kann durch Halogen), durch einen Heteroarylrest aus der Reihe Pyrralyl, Pyridyl, Pyridyl-N-oxid, Pyrimidinyl, Pyrazolyl, Thiazolyl Furanyl, Thienyl, Triazolyl, Oxadiazolyl, Thiadiazolyl, Pyrazinyl, Triazinyl und Isochinolinyl (welcher selbst wiederum substituiert sein kann durch Halogen, Nitro, C₁-C₄-Alkyl, C₁-C₄-Halogenalkyl, C₁-C₄-Alkoxy, C₃-C₆-Cycloalkyl) oder durch einen Heteroarylalkylrest aus der Reihe Triazolyl-C₁-C₄-alkyl, Pyridyl-C₁-C₄-alkyl, Pyrimidinyl-C₁-C₄-alkyl und Oxadiazolyl-C₁-C₄-alkyl (welcher selbst wiederum substituiert sein kann durch C₁-C₄-Alkyl).

5. Mittel, **gekennzeichnet durch** einen Gehalt von mindestens einer Verbindung der Formel (I) gemäß einem der Ansprüche 1 bis 4.

6. Nichttherapeutische Verfahren zum Bekämpfen von Schädlingen **dadurch gekennzeichnet, dass** man eine Verbindung der Formel (I) gemäß Anspruchen 1 bis 4 oder ein Mittel germäß Anspruch 5 auf die Schädlinge und/oder ihren Lebensraum einwirken lässt.

## Claims

1. Compounds of the formula (I) 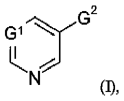 in which
G¹ represents N, CH or C-Halogen,
G² represents 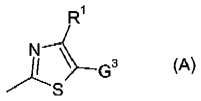 in which
R¹ represents a radical from the group consisting of cyano, nitro, fluorine, chlorine, bromine and iodine,
G³ represents a herocycle from the group consisting of dioxanyl, oxazolinyl, dihydrooxadiazinyl, dihydrodioxazinyl and hydroxypyndyl which is substituted by one or more radicals from the group consisting of halogen, cyano, nitro, alkyl, haloalkyl, cycloalkyl, alkoxy, haloalkoxy, alkylthio, haloalkylthio, alkylsulphinyl, alkylsulphonyl, haloalkylsulphinyl, haloalkylsulphonyl, amino, alkylamino, dialkylamino, alkylcarbonylamino, alkoxycarbonylamino, alkoxyalkyl, haloalkoxyalkyl, alkenyl, alkynyl, cycloalkylalkyl, alkylcarbonyl, alkoxycarbonyl, ammocarbonyl, pyndyl and pyrimidyl,
G³ also represents heteroaryl from the group consisting of pyrrolyl pyrazolyl, imidazolyl, 1,2,3-triazolyl, 1,2,4-triazolyl, oxazolyl, isoxazolyl, thiazolyl, isothiazolyl, 1,2,3-oxadiazolyl, 1,2,4-oxadiazolyl, 1,3,4-oxadiazolyl, 1,2,5-oxadiazolyl, 1,2,3-thiadiazolyl, 1,2,4-thiadiazolyl, 1,3,4-thiadiazolyl, 1,2,5-thiadiazolyl, pyridyl, pyrimidinyl, pyridazinyl, pyrazinyl, 1,2,3-triazinyl, 1,2,4-triazinyl, 1,3,5-triazinyl, benzofuryl, benzisofuryl, benzothienyl, benzisothienyl, indolyl, isoindolyl, indazolyl, benzothiazolyl, benzisothiazolyl, benzoxazolyl, benzisoxazolyl, benzimidazolyl, 2,1,3-benzoxadiazole, quinolinyl, isoquinolinyl, cinnolinyl, phthalazinyl, quinazolinyl, quinoxalinyi, naphthyridinyl, benzotriazinyl, purinyl, pteridinyl and indolizinyl which is substituted by a heterocyclyl radical from the group consisting of triazolinonyl, dihydrodioxazinyl, dihydrooxadiazinyl, dioxolanyl, dioxanyl (in particular 1,3-dioxan-2-yl), piperidinonyl, pyrrolidinonyl and pyrazolinonyl (which for its part may be substituted by alkyl and haloalkyl), by phenyl (which for its part may be substituted by halogen, cyano, nitro, alkyl and haloalkyl), by a heteroaryl radical from the group consisting of pyrrolyl pyridyl, pyridyl N-oxide, pyrimidyl, imidazolyl, pyrazolyl, oxazolyl, thiazolyl, furanyl, thienyl, triazolyl, oxadiazolyl, thiadiazolyl, pyrazinyl, triazinyl, tetrazinyl and isoquinolinyl (which for its part may be substituted by halogen, nitro, alkyl, haloalkyl, alkoxy, haloalkoxy, alkoxyalkyl, alkylthio, alkylthioalkyl and cycloalkyl) or by a heteroarylalkyl radical from the group consisting of triazolylalkyl, pyridylalkyl, pyrimidylalkyl and oxadiazolylalkyl (which for its part may be substituted by alkyl),
and also salts, metal complexes and N-oxides of the compounds of the formula (I).

2. Compounds of the formula (I) according to Claim 1 in which
G¹ represents N, CH or C-halogen,
G² represents 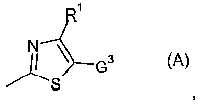 in which
R¹ represents cyano, fluorine or chlorine,
G³ represents a heterocycle from the group consisting of 1,3-dioxan-2-yl, oxazolinyl, dihydrooxadiazinyl dihydrodioxazinyl and hydroxypyridyl which is substituted by one or more radicals from the group consisting of halogen, cyano, nitro, C₁-C₆-alkyl, C₁-C₆-haloalkyl, C₃-C₆-cycloalkyl, C₁-C₆-alkoxy, C₁-C₆-haloalkoxy, C₁-C₆-alkylthio, C₁-C₆-haloaylthio, C₁-C₆-alkylsulphinyl, C₁-C₆-alkylsulphonyl, C₁-C₆. haloalkylsulphinyl, C₁-C₆-haloalkylsulphonyl, amino, C₁-C₆-alkylamino, di-(C₁-C₆-alkyl)amino, C₁-C₆-alkylcarbonylamino, C₁-C₆-alkoxycarbonylamino, C₁-C₆-alkoxy-C₁-C₆-alkyl, C₁-C₆-haloalkoxy-C₁-C₆-alkyl, C₂-C₆-alkenyl, C₂-C₆-alkynyl, C₃-C₆-cycloalkyl-C₁-C₆-alkyl, C₁-C₆-alkylcarbonyl, C₁-C₆-alkoxycarbonyl, aminocarbonyl, pyridyl and pyrimidyl,
G³ also represents Heteroaryl from the group consisting of pyrrolyl, pyrazolyl, imidazolyl, 1,2,3-triazolyl, 1,2,4-triazolyl, oxazolyl, isoxazolyl, thiazolyl, isothiazolyl, 1,2,3-oxadiazolyl, 1,2,4-oxadiazolyl, 1,3,4-oxadiazolyl, 1,2,5-oxadiazolyl, 1,2,3-thiadiazolyl, 1,2,4-thiadiazolyl, 1,3,4-thiadiazolyl, 1,2,5-thiadiazolyl, pyridyl, pyrimidinyl, pyridazinyl, pyrazinyl, 1,2,3-triazinyl, 1,2,4-triazinyl, 1,3,5-triazinyl, benzofuryl, bensofuryl, benzothienyl, benzisothienyl, indolyl, isoindolyl, indazolyl, benzothiazolyl, benzisothiazolyl, benzoxazolyl, benzisoyazolyl, benzimidazolyl, 2,1,3-benzoxadiazole, quinolinyl, isoquinolinyl, cinnolinyl, phthalazinyl, quinazolinyl, quinoxalinyl, naphthyridinyl, benzotriazinyl, purinyl, pteridinyl and indolizinyl, each of which is substituted by a heterocyclyl radical from the group consisting of morpholinyl, triazolinonyl, dihydrodioxazinyl, dihydrooxadiazinyl, dioxolanyl, dioxanyl piperidinonyl, pyrrolidinonyl and pyrazolinonyl (which for its part may be substituted by C₁-C₆-alkyl and C₁-C₆-haloakyl), by phenyl (which for its part may be substituted by halogen, cyano, nitro, C₁-C₆-alkyl and C₁-C₆-haloalkyl), by a heteroaryl radical from the group consisting of pyrrolyl, pyridyl, pyridyl N-oxide, pyrimidyl, imidazolyl, pyrazolyl, oxazolyl, thiazolyl, furanyl, thienyl, triazolyl, oxadiazolyl, thiadiazolyl, pyrazinyl, triazinyl, tetrazinyl and isoquinolinyl (which for its part may be substituted by halogen, nitro, C₁-C₆-alkyl, C₁-C₆-haloakyl, C₁-C₆-alkoxy, C₁-C₆-haloalkoxy, C₁-C₆-alkoxy-C₁-C₆-alkyl, C₁-C₆-alkylthio, C₁-C₆-akylthio-C₁-C₆-alkyl and C₃-C₆-cycloalkyl) or by a heteroarylalkyl radical from the group consisting of triazolyl-C₁-C₆-alkyl, pyridyl-C₁-C₆-alkyl, pyrimidyl-C₁-C₆-alkyl and oxadiazolyl-C₁-C₆-alkyl (which for its part may be substituted by C₁-C₆-alkyl).

3. Compounds of the formula (I) according to Claim 1 in which
G¹ represents N, CH or C-halogen,
G² represents 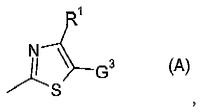 in which
R¹ represents cyano, fluorine or chlorine,
G³ represents a heterocycle from the group consisting of 1,3-dioxan-2-yl, oxazolinyl, dihydrooxadiazinyl, dihydrodioxazinyl and hydroxypyridyl which is substituted by one or more radicals from the group consisting of halogen, cyano, nitro, C₁-C₄-alkyl, C₁-C₄-haloalkyl, C₃-C₆-cycloalkyl, C₁-C₄-alkoxy, C₁-C₄-haloalkoxy, C₁-C₄-alkylthio, C₁-C₄-haloalkylthio, C₁-C₄-alkylsulphinyl, C₁-C₄-alkylsulphonyl, C₁-C₄-haloalkylsulphinyl, C₁-C₄-haloalkylsulphonyl, amino, C₁-C₄-alkylamino, di-(C₁-C₄-alkyl)amino, C₁-C₄-alkylcarbonylamino, C₁-C₄-alkoxycarbonylamino, C₁-C₄-alkoxy-C₁-C₄-alkyl, C₁-C₄-haloalkoxy-C₁-C₄-alkyl, C₂-C₄-alkenyl, C₂-C₄-alkynyl, C₃-C₆-cycloalkyl-C₁-C₄-alkyl, C₁-C₄-alkylcarbonyl, C₁-C₄-alkoxycarbonyl, aminocarbonyl, pyridyl and pyrimidyl,
G³ also represents a heteroaryl radical from the group consisting of pyrrolyl, pyrazolyl, imidazolyl, 1,2,3-triazolyl, 1,2,4-triazolyl, oxazolyl, isoxazolyl, thiazolyl, isothiazolyl, 1,2,3-oxadiazolyl, 1,2,4-oxadiazolyl, 1,3,4-oxadiazolyl, 1,2,5-oxadiazolyl, 1,2,3-thiadiazolyl, 1,2,4-thiadiazolyl, 1,3,4-thiadiazolyl, 1,2,5-thiadiazolyl, pyridyl, pyrimidinyl, pyridazinyl, pyrazinyl 1,2,3-triazinyl, 1,2,4-triazinyl, 1,3,5-triazinyl, benzofuryl, benzisofuryl, benzothienyl, benzisothienyl, indolyl, isoindolyl, indazolyl, benzothiazolyl, benzisothiazolyl, benzoxazolyl, benzisoxazolyl, benzimidazolyl, 2,1,3-benzoxadiazole, quinolinyl, isoquinolinyl, cinnolinyl, phthalazinyl, quinazolinyl, quinoxalinyl, naphthyridinyl, benzotriazinyl, purinyl, pteridinyl and indolizinyl which is substituted by a heterocyclyl radical from the group consisting of morpholinyl, triazolinonyl, dihydrodioxazinyl, dihydrooxadiazinyl, dioxolanyl, dioxanyl, piperidinonyl, pyrrolidinonyl and pyrazolinonyl (which for its part may be substituted by C₁-C₄-alkyl and C₁-C₄-haloalkyl), by phenyl (which for its part may be substituted by halogen, cyano, nitro, C₁-C₄-alkyl and C₁-C₄-haloalkyl), by a heteroaryl radical from the group consisting of pyrrolyl, pyridyl, pyridyl N-oxide, pyrimidyl, imidazolyl, pyrazolyl, oxazolyl, thiazolyl, furanyl, thienyl, triazolyl, oxadiazolyl, thiadiazolyl, pyrazinyl, triazinyl, tetrazinyl and isoquinolinyl (which for its part may be substituted by halogen, nitro, C₁-C₄-alkyl, C₁-C4-haloalkyl, C₁-C₄-alkoxy, C₁-C₄-haloalkoxy, C₁-C₄-alkoxy-C₁-C₄-alkyl, C₁-C₄-alkylthio, C₁-C₄-alkylthio-C₁-C₄-alkyl and C₃-C₆-cycloalkyl) or by a heteroarylalkyl radical from the group consisting of triazolyl-C₁-C₄-alkyl, pyridyl-C₁-C₄-alkyl, pyrimidyl-C₁-C₄-alkyl and oxadiazolyl-C₁-C₄-alkyl (which for its part may be substituted by C₁-C₄-alkyl).

4. Compounds of the formula (I) according to Claim 1
in which
G¹ represents N, CH or C-halogen, G² represents 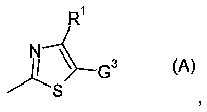 in which
R¹ represents fluorine or chlorine,
G³ also represents a heteroaryl radical from the group consisting of pyrazolyl, 1,2,4-oxadiazolyl, pyridyl, pyrimidinyl, 1,3,5-triazinyl, triazinyl and oxadiazolyl which is substituted by a heterocyclyl radical from the group consisting of morpholinyl, triazolinonyl, dihydrodioxazinyl, dihydrooxadiazinyl, piperidinonyl, pyrrolidinonyl and pyrazolinonyl (which for its part may be substituted by C₁-C₄-alkyl and C₁-C₄-haloalkyl), by phenyl (which for its part may be substituted by halogen), by a heteroaryl radical from the group consisting of pyrrolyl, pyridyl, pyridyl N-oxide, pyrimidinyl, pyrazolyl, thiazolyl, furanyl, thienyl, triazolyl, oxadiazolyl, thiadiazolyl, pyrazinyl, triazinyl und isoquinolinyl (which for its part may be substituted by Halogen, nitro, C₁-C₄-alkyl, C₁-C₄-haloalkyl, C₁-C₄-alkoxy, C₃-C₆-cycloalkyl) or by a heteroarylalkyl radical from the group consisting of triazolyl-C₁-C₄-alkyl, pyridyl-C₁-C₄-alkyl, pyrimidinyl-C₁-C₄-alkyl and oxadiazolyl-C₁-C₄-alkyl (which for its part may be substituted by C₁-C₄-alkyl).

5. Composition, **characterized in that** it comprises at least one compound of the formula (I) according to any of Claims 1 to 4.

6. Nontherapeutic method for controlling pests, **characterized in that** a compound of the formula (I) according to any of Claims 1 to 4 or a composition according to Claim 5 is allowed to act on the pests and/or their habitat.

## Revendications

1. Composés de formula (I) 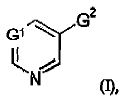 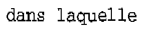
G¹ représente N, CH ou C-halogène,
G² représente 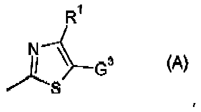 avec
R¹ représentant un radical de la série cyano, nitro, fluor, chlore, brome et iode,
G³ représentant un hétérocycle de la série dioxanyle, oxazolinyle, dihydrooxadiazinyle, dihydrodioxazinyle et hydroxypyridyle, qui est substitué par un ou plusieurs radicaux de la série halogène, cyano, nitro, alkyle, halogénoalkyle, cycloalkyle, alcoxy, halogénoalcoxy, alkylthio, haloalkylthio, alkylsulfinyle, alkylsulfonyle, haloalkylsulfinyle, haloalkylsulfonyle, amino, alkylamino, dialkylamino, alkylcarbonylamino, alcoxycarbonylamino, alcoxyalkyle, halogénoalcoxyalkyle, alcényle, alcynyle, cycloalkylalkyle, alkylcarbonyle, alcoxycarbonyle, aminocarbonyle, pyridyle et pyrimidyle,
G³ représentant également un hétéroaryle de la série pyrrolyle, pyrazolyle, imidazolyle, 1,2,3-triazolyle, 1,2,4-triazolyle, oxazolyle, isoxazolyle, thiazolyle, isothiazolyle, 1,2,3-oxadiazolyle, 1,2,4-oxadiazolyle, 1,3,4-oxadiazolyle, 1,2,5-oxadiazolyle, 1,2,3-thiadiazolyle, 1,2,4-thiadiazolyle, 1,3,4-thiadiazolyle, 1,2,5-thiadiazolyle, pyridyle, pyrimidinyle, pyridazinyle, pyrazinyle, 1,2,3-triazinyle, 1,2,4-triazinyle, 1,3,5-triazinyle, benzofuryle, benzisofuryle, benzothiényle, benzisothiényle, indolyle, isoindolyle, indazolyle, benzothiazolyle, benzisothiazolyle, benzoxazolyle, benzisoxazolyle, benzimidazolyle, 2,1,3-benzoxadiazole, quinolinyle, isoquinolinyle, cinnolinyle, phtalazinyle, quinazolinyle, quinoxalinyle, naphtyridinyle, benzotriazinyle, purinyle, ptéridinyle et indolizinyle, qui est substitué par un radical hétérocyclyle de la série triazolinonyle, dihydrodioxazinyle, dihydrooxadiazinyle, dioxolanyle, dioxanyle (notamment 1,3-dioxan-2-yle), pipéridinonyle, pyrrolidinonyle et pyrazolinonyle (qui peut lui-même être substitué par alkyle et halogénalkyle), par phényle (qui peut lui-même être substitué par halogène, cyano, nitro, alkyle et halogénoalkyle), par un radical hétéroaryle de la série pyrrolyle, pyridyle, pyridyl-N-oxyde, pyrimidyle, imidazolyle, pyrazolyle, oxazolyle, thiazolyle, furanyle, thiényle, triazolyle, oxadiazolyle, thiadiazolyle, pyrazinyle, triazinyle, tétrazinyle et isoquinolinyle (qui peut lui-même être substitué par halogène, nitro, alkyle, halogénoalkyle, alcoxy, halogénoalcoxy, alcoxyalkyle, alkylthio, alkylthioalkyle et cycloalkyle) ou par un radical hétéroarylalkyle de la série triazolylalkyle, pyridylalkyle, pyrimidylalkyle et oxadiazolylalkyle (qui peut lui-même être substitué par alkyle),
ainsi que les sels, complexes métalliques et N-oxydes des composés de formule (I).

2. Composés de formule (I) selon la revendication 1, dans lesquels
G¹ représente N, CH ou C-halogène,
G² représente 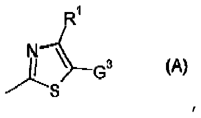 avec
R¹ représentant cyano, fluor ou chlore,
G³ représentant un hétérocycle de la série 1,3-dioxan-2-yle, oxazolinyle, dihydrooxadiazinyle, dihydrodioxazinyle et hydroxypyridyle, qui est substitué par un ou plusieurs radicaux de la série halogène, cyano, nitro, alkyle en C₁-C₆, halogénoalkyle en C₁-C₆, cycloalkyle en C₃-C₆, alcoxy en C₁-C₆, halogénoalcoxy en C₁-C₆, alkylthio en C₁-C₆, haloalkylthio en C₁-C₆, alkylsulfinyle en C₁-C₆, alkylsulfonyle en C₁-C₆, haloalkylsulfinyle en C₁-C₆, haloalkylsulfonyle en C₁-C₆, amino, alkylamino en C₁-C₆, di(alkyle en C₁-C₆)amino, alkylcarbonylamino en C₁-C₆, alcoxycarbonylamino en C₁-C₆, alcoxy en C₁-C₆-alkyle en C₁-C₆, halogénoalcoxy en C₁-C₆-alkyle en C₁-C₆, alcényle en C₂-C₆, alcynyle en C₂-C₆, cycloalkyle en C₃-C₆-alkyle en C₁-C₆, alkylcarbonyle en C₁-C₆, alcoxycarbonyle en C₁-C₆, aminocarbonyle, pyridyle et pyrimidyle,
G³ représentant également un hétéroaryle de la série pyrrolyle, pyrazolyle, imidazolyle, 1,2,3-triazolyle, 1,2,4-triazolyle, oxazolyle, isoxazolyle, thiazolyle, isothiazolyle, 1,2,3-oxadiazolyle, 1,2,4-oxadiazolyle, 1,3,4-oxadiazolyle, 1,2,5-oxadiazolyle, 1,2,3-thiadiazolyle, 1,2,4-thiadiazolyle, 1,3,4-thiadiazolyle, 1,2,5-thiadiazolyle, pyridyle, pyrimidinyle, pyridazinyle, pyrazinyle, 1,2,3-triazinyle, 1,2,4-triazinyle, 1,3,5-triazinyle, benzofuryle, benzisofuryle, benzothiényle, benzisothiényle, indolyle, isoindolyle, indazolyle, benzothiazolyle, benzisothiazolyle, benzoxazolyle, benzisoxazolyle, benzimidazolyle, 2,1,3-benzoxadiazole, quinolinyle, isoquinolinyle, cinnolinyle, phtalazinyle, quinazolinyle, quinoxalinyle, naphtyridinyle, benzotriazinyle, purinyle, ptéridinyle et indolizinyle, qui est à chaque fois substitué par un radical hétérocyclyle de la série morpholinyle, triazolinonyle, dihydrodioxazinyle, dihydrooxadiazinyle, dioxolanyle, dioxanyle, pipéridinonyle, pyrrolidinonyle et pyrazolinonyle (qui peut lui-même être substitué par alkyle en C₁-C₆ et halogénalkyle en C₁-C₆), par phényle (qui peut lui-même être substitué par halogène, cyano, nitro, alkyle en C₁-C₆ et halogénoalkyle en C₁-C₆), par un radical hétéroaryle de la série pyrrolyle, pyridyle, pyridyl-N-oxyde, pyrimidyle, imidazolyle, pyrazolyle, oxazolyle, thiazolyle, furanyle, thiényle, triazolyle, oxadiazolyle, thiadiazolyle, pyrazinyle, triazinyle, tétrazinyle et isoquinolinyle (qui peut lui-même être substitué par halogène, nitro, alkyle en C₁-C₆, halogénoalkyle en C₁-C₆, alcoxy en C₁-C₆, halogénoalcoxy en C₁-C₆, alcoxy en C₁-C₆-alkyle en C₁-C₆, alkylthio en C₁-C₆, alkylthio en C₁-C₆-alkyle en C₁-C₆ et cycloalkyle en C₃-C₆) ou par un radical hétéroarylalkyle de la série triazolyl-alkyle en C₁-C₆, pyridyl-alkyle en C₁-C₆, pyrimidyl-alkyle en C₁-C₆ et oxadiazolyl-alkyle en C₁-C₆ (qui peut lui-même être substitué par alkyle en C₁-C₆).

3. Composés de formule (I) selon la revendication 1, dans lesquels
G¹ représente N, CH ou C-halogène,
G² représente 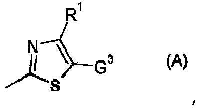 avec
R¹ représentant cyano, fluor ou chlore,
G³ représentant un hétérocycle de la série 1,3-dioxan-2-yle, oxazolinyle, dihydrooxadiazinyle, dihydrodioxazinyle et hydroxypyridyle, qui est substitué par un ou plusieurs radicaux de la série halogène, cyano, nitro, alkyle en C₁-C₄, halogénoalkyle en C₂-C₄, cycloalkyle en C₃-C₆, alcoxy en C₁-C₄, halogénoalcoxy en C₁-C₄, alkylthio en C₁-C₄, haloalkylthio en C₁-C₄, alkylsulfinyle en C₁-C₄, alkylsulfonyle en C₁-C₄, haloalkylsulfinyle en C₁-C₄, haloalkylsulfonyle en C₁-C₄, amino, alkylamino en C₁-C₄, di(alkyle en C₁-C₄)amino, alkylcarbonylamino en C₁-C₄, alcoxycarbonylamino en C₁-C₄, alcoxy en C₁-C₄-alkyle en C₁-C₄, halogénoalcoxy en C₁-C₄-alkyle en C₁-C₄, alcényle en C₂-C₄, alcynyle en C₂-C₄, cycloalkyle en C₃-C₆-alkyle en C₁-C₄, alkylcarbonyle en C₁-C₄, alcoxycarbonyle en C₁-C₄, aminocarbonyle, pyridyle et pyrimidyle,
G³ représentant également un radical hétéroaryle de la série pyrrolyle, pyrazolyle, imidazolyle, 1,2,3-triazolyle, 1,2,4-triazolyle, oxazolyle, isoxazolyle, thiazolyle, isothiazolyle, 1,2,3-oxadiazolyle, 1,2,4-oxadiazolyle, 1,3,4-oxadiazolyle, 1,2,5-oxadiazolyle, 1,2,3-thiadiazolyle, 1,2,4-thiadiazolyle, 1,3,4-thiadiazolyle, 1,2,5-thiadiazolyle, pyridyle, pyrimidinyle, pyridazinyle, pyrazinyle, 1,2,3-triazinyle, 1,2,4-triazinyle, 1,3,5-triazinyle, benzofuryle, benzisofuryle, benzothiényle, benzisothiényle, indolyle, isoindolyle, indazolyle, benzothiazolyle, benzisothiazolyle, benzoxazolyle, benzisoxazolyle, benzimidazolyle, 2,1,3-benzoxadiazole, quinolinyle, isoquinolinyle, cinnolinyle, phtalazinyle, quinazolinyle, quinoxalinyle, naphtyridinyle, benzotriazinyle, purinyle, ptéridinyle et indolizinyle, qui est substitué par un radical hétérocyclyle de la série morpholinyle, triazolinonyle, dihydrodioxazinyle, dihydrooxadiazinyle, dioxolanyle, dioxanyle, pipéridinonyle, pyrrolidinonyle et pyrazolinonyle (qui peut lui-même être substitué par alkyle en C₁-C₄ et halogénalkyle en C₁-C₄), par phényle (qui peut lui-même être substitué par halogène, cyano, nitro, alkyle en C₁-C₄ et halogénoalkyle en C₁-C₄), par un radical hétéroaryle de la série pyrrolyle, pyridyle, pyridyl-N-oxyde, pyrimidyle, imidazolyle, pyrazolyle, oxazolyle, thiazolyle, furanyle, thiényle, triazolyle, oxadiazolyle, thiadiazolyle, pyrazinyle, triazinyle, tétrazinyle et isoquinolinyle (qui peut lui-même être substitué par halogène, nitro, alkyle en C₁-C₄, halogénoalkyle en C₁-C₄, alcoxy en C₁-C₄, halogénoalcoxy en C₁-C₄, alcoxy en C₁-C₄-alkyle en C₁-C₄, alkylthio en C₁-C₄, alkylthio en C₁-C₄-alkyle en C₁-C₄ et cycloalkyle en C₃-C₆) ou par un radical hétéroarylalkyle de la série triazolyl-alkyle en C₁-C₄, pyridyl-alkyle en C₁-C₄, pyrimidyl-alkyle en C₁-C₄ et oxadiazolyl-alkyle en C₁-C₄ (qui peut lui-même être substitué par alkyle en C₁-C₄).

4. Composés de formule (I) selon la revendication 1, dans lesquels
G¹ représente N, CH ou C-halogène,
G² représente 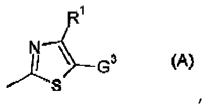 avec
R¹ représentant fluor ou chlore,
G³ représentant un radical hétéroaryle de la série pyrazolyle, 1,2,4-oxadiazolyle, pyridyle, pyrimidinyle, 1,3,5-triazinyle, triazinyle et oxadiazolyle, qui est substitué par un radical hétérocyclyle de la série morpholinyle, triazolinonyle, dihydrodioxazinyle, dihydrooxadiazinyle, pipéridinonyle, pyrrolidinonyle et pyrazolinonyle (qui peut lui-même être substitué par alkyle en C₁-C₄ et halogénalkyle en C₁-C₄), par phényle (qui peut lui-même être substitué par halogène), par un radical hétéroaryle de la série pyrrolyle, pyridyle, pyridyl-N-oxyde, pyrimidinyle, pyrazolyle, thiazolyle, furanyle, thiényle, triazolyle, oxadiazolyle, thiadiazolyle, pyrazinyle, triazinyle et isoquinolinyle (qui peut lui-même être substitué par halogène, nitro, alkyle en C₁-C₄, halogénoalkyle en C₁-C₄, alcoxy en C₁-C₄, cycloalkyle en C₃-C₆) ou par un radical hétéroarylalkyle de la série triazolyl-alkyle en C₁-C₄, pyridyl-alkyle en C₁-C₄, pyrimidinyl-alkyle en C₁-C₄ et oxadiazolyl-alkyle en C₁-C₄ (qui peut lui-même être substitué par alkyle en C₁-C₄).

5. Agent, **caractérisé par** une teneur en au moins un composé de formule (I) selon l'une quelconque des revendications 1 à 4.

6. Procédé non thérapeutique pour lutter contre des animaux nuisibles, **caractérisé en ce qu'**un composé de formule (I) selon les revendications 1 à 4 ou un agent selon la revendication 5 est laissé agir sur les animaux nuisibles et/ou leur habitat.
